# EUROPÄISCHE PATENTSCHRIFT

(11) **EP 0 915 340 B1**
(45) Veröffentlichungstag und Bekanntmachung des Hinweises auf die Patenterteilung: **04.01.2006**
(21) Anmeldenummer: 98118971.5
(22) Anmeldetag: 07.10.1998
(51) Int. Cl.: G01N 33/86, C12Q 1/56

(54) **Verfahren zur Bestimmung des antikoagulatorischen Potentials einer Probe und Verfahren zur Bestimmung der Glykosilierung von Thrombomodulin**
Process for the determination of the anticoagulant potential of a sample and process for the determination of the glycosylation of thrombomodulin
Procédé pour déterminer le potentiel anticoagulatoire d'un échantillon et procédé pour déterminer la glycosylation de la thrombomoduline

(30) Priorität: 07.11.1997 DE 19749197
(43) Veröffentlichungstag der Anmeldung: 12.05.1999
(73) Patentinhaber: Dade Behring Marburg GmbH, 35001 Marburg (DE)
(72) Erfinder: Kraus, Michael, Dr., 35041 Marburg (DE)

(56) Entgegenhaltungen:
- EP-A- 0 445 304
- EP-A- 0 488 317
- WO-A-92/03149
- FR-A- 2 689 640
- US-A- 5 051 357
- US-A- 5 525 478
- M-C BOURIN: "Effect of Rabbit Thrombomodulin on Thrombin Inhibition by Antithrombin in the Presence of Heparin" THROMBOSIS RESEARCH, Bd. 54, Nr. 1, 1989, Seiten 27-39, XP002094973
- K T PREISSNER, T KOYAMA, D MÜLLER, J TSCHOPP, G MÜLLER-BERGHAUS: "Domain Structure of the Endothelial Cell Receptor Thrombomodulin as Deduced from Modulation of Its Anticoagulant Functions" JOURNAL OF BIOLOGICAL CHEMISTRY, Bd. 265, Nr. 9, 25. März 1990, Seiten 4915-4922, XP002094974
- MARUYAMA I ET AL: "HUMAN THROMBOMODULIN IS NOT AN EFFICIENT INHIBITOR OF THE PROAGULANT ACTIVITY OF THROMBIN" JOURNAL OF CLINICAL INVESTIGATION, Bd. 75, Nr. 3, März 1985, Seiten 987-991, XP000123084
- Ohishi R. et al., Thrombosis and Haemostasis, 70(3), 1993, 423-426
- TAKAHASHI Y; ET AL, - SOLUBLE THROMBOMODULIN PURIFIED FROM HUMAN URINE EXHIBITS A POTENT ANTICOAGULANT EFFECT IN VITRO AND IN VIVO, 1995, THROMBOSIS AND HAEMOSTASIS, VOL - 73 *r*n NR - 5,PG - 805-811
- TAKAHASHI Y; ET AL, - SOLUBLE THROMBOMODULIN PURIFIED FROM HUMAN URINE EXHIBITS A POTENT ANTICOAGULANT EFFECT IN VITRO AND IN VIVO, 1995, THROMBOSIS AND HAEMOSTASIS, VOL - 73 NR - 5,PG - 805-811
- OHISHI R. ET AL: "EVIDENCE THAT THE PROTEIN C ACTIVATION PATHWAY AMPLIFIES THE INHIBITION OF THROMBIN GENERATION BY RECOMBINAT HUMAN THROMBOMODULIN IN PLASMA" THROMBOSIS AND HAEMOSTASIS, Bd. 70, Nr. 3, 1993, Seiten 423-426,
- Ohishi R. et al: Thrombosis and Haemostasis, 70(3), 1993, 423-426
- TAKAHASHI Y. ET AL: 'SOLUBLE THROMBOMODULIN PURIFIED FROM HUMAN URINE EXHIBITS A POTENT ANTICOAGULANT EFFECT IN VITRO AND IN VIVO', 1995, THROMBOSIS AND HAEMOSTASIS, VOL 73, NR 5, PG 805-811

## Beschreibung

Die Anmeldung betrifft ein Verfahren zur Bestimmung des antikoagulatorischen Potentials einer Probe durch Zusatz von Thrombomodulin und Thromboplastin in einem Gerinnungstest.

Die Aktivierung der Gerinnung mündet in der Umsetzung des Proenzyms Prothrombin zur aktiven Protease Thrombin. Thrombin beschleunigt seine Entstehung selbst, in dem es die Kofaktoren Faktor V und Faktor VIII durch proteolytische Spaltung aktiviert. Diese aktivierten Kofaktoren bilden mit den Proteasen Faktor Xa bzw. IXa aktive Enzym/Kofaktor-Komplexe auf Phospholipidoberflächen, deren Aktivität um ca. den Faktor 10.000 höher ist als die der singulären Proteasen. Durch diese positive Rückkopplung kommt es quasi explosionsartig zur Entstehung großer Mengen an Thrombin. Thrombin setzt Fibrinogen zu Fibrin um, das im Normalfall zum Wundverschluß und zur Wundheilung führt. Um eine lebensbedrohende Ausweitung der Gerinnung zu verhindern, die zu einem Verschluß des Gefäßsystems im Körper, also zu Thrombosen führen würden, müssen sowohl die aktive Protease als auch die Nachlieferung der Protease unterbunden werden. Aktive Proteasen werden im Körper durch Protease-Inhibitoren durch die Ausbildung kovalenter Komplexe neutralisiert. Der wichtigste Protease-Inhibitor ist das Antithrombin III, dessen antikoagulatorische Wirkung durch Heparinsulfate beschleunigt wird. Die fortgesetzte Entstehung aktiver Gerinnungsproteasen wird durch Thrombin selbst rückkoppelnd unterbrochen. Thrombin bindet an das Membranprotein Thrombomodulin und verliert dadurch seine prokoagulatorischen Eigenschaften wie die Aktivierung von Plättchen oder die Umsetzung von Fibrinogen. Der Thrombin/Thrombomodulin-Komplex setzt das Proenzym Protein C in Anwesenheit von Calcium-lonen zur aktiven Protease Protein Ca (APC) um (Wirkung A). Daneben wirkt Thrombomodulin, ein Heparansulfat, durch seine Glykosylierung selbst antikoagulatorisch. Die Bildung eines inaktiven Thrombin-Antithrombin III Komplexes wird dadurch beschleunigt (Dittman WA, Majerus PW, Blood 1990; 75: 329-336; Bourin M-C, Lindahl U, Biochem J 1990; 270: 419-425). Das entstandene APC bildet mit seinem Kofaktor Protein S einen Komplex, der die aktiven Kofaktoren Faktor VIIIa und Faktor Va proteolytisch spaltet und dadurch inaktiviert. APC unterbricht somit die starke Stimulierung durch diese Kofaktoren und die weitere Entstehung von der Faktoren Xa bzw. Thrombin. Ein weiteres Membranprotein, der endotheliale Protein C Rezeptor scheint die Protein C aktivierende Aktivität des Thrombin/Thrombomodulin-Komplexes zu stimulieren

Dieses oben beschriebene Protein C-System stellt einen wichtigen antikoagulatorischen Mechanismus dar. Dies wird dadurch bestätigt, daß Personen mit erblichen oder erworbenen Mängeln oder Defekten an Protein C oder Protein S mit hoher Wahrscheinlichkeit Thrombosen, insbesondere rezidivierende venöse Thrombosen, erleiden. Neben Protein C und Protein S können weitere Faktoren die Aktivität des Systems beeinflussen, so von Willebrand Faktor und Faktor IXa , die Faktor VIIIa vor proteolytischem Abbau schützen können. Erworbene Störungen können auch auf die Entstehung von Lupus Antikoagulantien zurückgehen. Dies sind gegen Phospholipide gerichtete Antikörper, die die zur Funktion notwendige Anbindung der Protease/Kofaktor Komplexe an Phospholipid-Oberflächen stören. Weiterhin wurde eine Mutation des Faktors V beschrieben, der nicht mehr oder zumindest nur sehr schlecht durch APC inaktiviert werden kann. Des weiteren sind Mutationen der am Thrombin/Thrombomodulin-Komplex beteiligten Faktoren bekannt, die zu einer verringerten Bildung von aktiviertem Protein C führen, wie Mutationen des Thrombomodulins selbst , des Protein C und des Thrombins.

Defekte oder Mangel des Antithrombin III sind eine weitere wichtige Ursache für die Entstehung von Thrombosen. Die Bestimmung des Antithrombin III erfolgt in der Regel durch Zusatz von Thrombin und Heparin zu einer hoch verdünnten Probe und Bestimmung des residualen Thrombins durch Zusatz eines chromogenen Substrates oder von Fibrinogen und der Bestimmung der Umsatzrate bzw. der Bildung einer Fibringerinnsels.

Aufgrund der vielen möglichen Störungen des für seine antithrombotische Wirkung wichtigen Protein C Systems, ist es in der klinischen Diagnostik sinnvoll, einen Screening Test anzuwenden, der eine Störung in diesem System, d.h. seines antikoagulatorischen Potentials generell anzeigt. Dies gilt insbesondere dann, wenn bestimmte Störungen, wie in diesem Fall durch von Willebrand-Faktor, Faktor IXa, Lupus Antikoagulant oder die Mutation des Faktors V, nur unter großem Aufwand in speziell darin erfahrenen Labors analysiert werden können. Darüber hinaus kann ein Screening Test zur Erfassung des Protein C System Potentials auch Störungen anzeigen, deren Ursachen im einzelnen nur schlecht aufzuklären sind, wie etwa der Einfluß von Akut Phase Reaktionen oder Entzündungen, da das Zusammenspiel verschiedener Faktoren aus einer Summe von Einzelfaktor-Bestimmungen nicht schlüssig zu eruieren ist. Weiterhin können in einem solchen Screening Test Störungen miterfaßt werden, deren Ursache derzeit noch nicht bekannt ist. Ein solcher Test dient damit zur Suche nach einzelnen oder mehreren Faktorstörungen eines Patienten, die zu einem erhöhten Thromboserisiko führen können.

Die Anwendung eines Testes der das antikoagulatorische Potential einer Probe, also des Protein C Systems und/oder von Antithrombin III erfaßt, geht über-die Bestimmung einer einzelnen Ursache hinaus und erhält eine eigene Wertigkeit, die in der klinischen Praxis zur Erkennung einer erhöhten Thromboseneigung (Thrombophilie) und damit Konsequenzen für die Therapie, wie beispielsweise der Antikoagulationstherapie mit Coumarin-Derivaten oder Heparinen, aufzeigt. Eine weitere Anwendung dieses Testes ist somit die Steuerung der Antikoagulations-Therapie.

Bisher wurden Protein C oder Protein S als Einzelfaktoren auf ihre Funktionalität untersucht. Hierzu wird die Probe oder aus der Probe isoliertes Protein C zunächst im Unterschuß zu einem Protein C-Mangelplasma zugesetzt. Die Aktivierung des Protein C erfolgt anschließend entweder durch Zusatz von Thrombin oder einer Kombination von Thrombin und Thrombomodulin oder durch Zugabe eines Schlangengifts von *Agkistrodon contortrix,* das unter seinem Handelsnamen Protac® (Fa. Pentapharm, Basel, Schweiz) bekannt ist. Die Detektion des in der Probe vorhanden Protein C erfolgt entweder anhand der Verlängerung der Gerinnungszeit durch den antikoagulatorischen Effekt des in der Probe vorhandenen Protein C oder durch Umsetzung eines für Thrombin spezfischen Substrats. Alternativ kann durch Verwendung eines spezifischen Substrats für APC die Protein C-Aktivität nach Aktivierung mit Thrombin oder Protac® auch direkt chromogen bestimmt werden.

Die Protein S Bestimmungen erfolgen durch Mischung der Probe mit PS-Mangelplasma. Die Bestimmung der antikoagulatorischen Wirkung von APC erfolgt durch Bestimmung der Gerinnungsverlängerung. Das hierzu benötigte APC wird entweder zugegeben oder mittels Protac® das Protein C im PS-Mangelplasma aktiviert (Bertina, RM, Res Clin Lab 1990; 20: 127-138). Ein Verfahren zur Bestimmung von Protein S unter Verwendung von Thrombomodulin wurde von Matschiner (US 5,525,478) beschrieben (siehe weiter unten).

Bekannte Verfahren zur Bestimmung von Protein C unter Verwendung von Thrombomodulin beruhen auf der Isolierung von Protein C aus der Probe mittels Adsorption. Dieses isolierte Protein C aus der Probe wird anschließend mittels Thrombin/Thrombomodulin Komplex aktiviert und das erzeugie, aktive Protein C im chromogenen Test nachgewiesen (Thiel, W. et al., Blut 1986; 52: 169-177). Dieses Verfahren ist kompliziert und erfaßt nicht das gesamte Potential des Protein C Systems. Darüberhinaus ist die Anwendung auf chromogene Verfahren beschränkt, d.h. die physiologische Auswirkung auf die Bildung eines Fibringerinnsels kann damit nicht erfaßt werden.

In der EP 0 711 838 wird ein Verfahren zur funktionellen Bestimmung von Varianten des Faktor V beschrieben, deren aktivierte Form gegenüber normalen Faktor Va sich durch eine verringerte Inaktivierung durch APC gekennzeichnet sind. Dazu wird die Probe mit einem Faktor V Mangelplasma gemischt um störende Einflüsse, etwa Faktorenmangel, Lupus Antikoagulantien oder therapeutische Einflüsse (orale Antikoagulation, Heparin) auszuschließen und anschließend in Gegenwart von aktiviertem Protein C ein Gerinnungstest durchgeführt.

Beschrieben wurde auch schon, daß ein Verfahren zum Nachweis von Thrombomodulin zum Nachweis von Mutanten des Thrombins eingesetzt wird, die dadurch gekennzeichnet sind, daß sie keinen aktiven Komplex mit Thrombomodulin bilden. Dazu wird die Probe verdünnt, so daß nach Aktivierung des Prothrombins zu Thrombin durch dem Fachmann an sich bekannte Enzyme aus Schlangengiften keine Gerinnsel entstehen, die die anschließende Bestimmung stören. Nach Zusatz von Thrombomodulin und Protein C wird die Entstehung von aktiviertem Protein C durch Umsetzung eines chromogenen Protein C Substrats verfolgt. Dieses Verfahren wurde auch schon verwendet um die Aktivität verschiedener natürlicher Varianten des Prothrombins respektive Thrombins mit Thrombomodulin zu prüfen.

Die bisher aufgeführten Methoden sind nur dazu geeignet Störungen des Protein C-Systems durch den jeweils singulär untersuchten Faktor zu detektieren. Sie eignen sich aufgrund der weiter vorne angestellten Überlegungen nicht als Screening Tests. Einer breiten Einführung als Screening Teste stand bisher auch die unzureichende Praktikabilität der Bestimmungsverfahren entgegen.

Zur Bestimmung von FV Störungen modifizierten Amer et al. (Thromb. Res. 1990; 57: 247-258) die aktivierte, partielle Thromboplastinzeit (APTT). Die APTT ist ein Standardverfahren zur Detektion von Gerinnungsstörungen, d.h. sie dient zur Erkennung von Blutungsneigungen. Nach Aktivierung des Probenplasmas mittels einer aktivierenden Oberfläche wird die Gerinnung nicht wie bei der APTT üblich durch Zugabe einer Calciumchlorid-Lösung gestartet, sondern gleichzeitig mit den Calciumionen wird APC mit zugegeben. Die Gerinnungszeiten werden durch die antikoagulatorische Wirkung des exogen zugegebenen APC verlängert. Dieser Test erkennt somit bereits viele Störungen des Protein C -Systems außer Defekten oder Mängeln des Protein C in der Probe, da APC exogen zugegeben wird, und auch keine Störungen, die etwa die Wechselwirkung von Protein C und/oder Thrombins mit Thrombomodulin betreffen.

In der DE 44 27 785 wird ein Verfahren beschrieben zur Erfassung von Störungen des Protein C Systems, in dem das Protein C der zu untersuchenden Probe (endogen) mit einem Protein C Aktivator zunächst voraktiviert wird. Anschließend wird die Wirkung des entstandenen APC auf die Verzögerung der Thrombinentstehung in einem Gerinnungstest überprüft. Als Protein C Aktivatoren kommen bekannte Aktivatoren wie Enzyme aus Schlangengiften (beispielsweise von *Agkistrodon contortrix,* Handelsname Protac®) oder Thrombin/Thrombomodulin-Komplexe zur Anwendung. Die Detektion der Thrombinentstehung kann über die Gerinnselbildung (klassisches Verfahren) oder mittels eines chromogenen Substrats erfolgen. Die Gerinnungsteste als Grundlage zur Erkennung der antikoagulatorischen Wirkung des Protein C Systems umfassen alle dem Fachmann an sich bekannte Verfahren wie die der APTT, der Thromboplastinzeit (PT), der Russell's Viper Venom Zeit (RVVT), dem Zusatz von aktivierten Gerinnungsfaktoren oder von Schlangengiften oder Enzymen daraus die letztlich zur Bildung von Thrombin und damit von aktiviertem Faktor V führen. In diesem Verfahren werden gegenüber bisherigen Verfahren alle Störungen des Protein C Systems mit Ausnahme von Varianten des Thrombins als auch des Thrombomodulins erfaßt. Weitere Varianten des Protein C können in diesem Verfahren, in dem präformierte Thrombin/Thrombomodulin-Komplexe verwendet werden, detektiert werden.

Basierend auf der Thromboplastinzeit, einem Standardverfahren in der Gerinnungsdiagnostik wird in der FR 2 689 640 ein Verfahren beschrieben, in dem in einer Probe durch Zugabe von Thromboplastin und Calcium die Gerinnung aktiviert wird. Das dabei entstehende Thrombin aktiviert bei gleichzeitiger Zugabe von Thrombomodulin das Protein C in der Probe (endogen). In Abhängigkeit von der Funktionstüchtigkeit des Protein C Systems wirkt APC der Entstehung von Thrombin entgegen. Nach 15 Minuten wird durch Komplexierung der Calciumionen die weitere Gerinnungsaktivität unterbrochen und das entstandene Thrombin durch Umsatz eines spezifischen, chromogenen Substrats bestimmt. Die entstandene Menge an Thrombin ist indirekt proportional zur Funktionsfähigkeit des Protein C Systems. Alle Störungen des Protein C Systems der Probe lassen sich erkennen, da sowohl das endogene Protein C, als auch das endogene Prothrombin aktiviert werden. Der Test hat jedoch einige Nachteile. Zum einen ist durch die lange Gesamtmeßzeit von 16 Minuten dieses Verfahren für einen routinegängigen Einsatz als Screening Test ungeeignet. Zum zweiten wird in der Probe ein Gerinnsel vor der eigentlichen Entstehung von aktiviertem Protein C erzeugt, so daß dieses Verfahren nur in Kombination mit chromogenen Meßmethoden möglich ist, die den Umsatz des erzeugten Thrombins erfassen. Die traditionelle Meßmethodik, die die Entstehung des Fibringerinnsels erfaßt ist damit nicht mehr möglich. Um Interferenzen mit dem entstehenden Gerinnsel zu vermeiden wird in dem Verfahren nach Duchemin et al. daher vor der Untersuchung das Fibrinogen der Probe entfernt, beispielsweise durch Zusatz von fibrinspaltenden Enzymen.

Ähnliche Verfahren werden von Rijkers et al. (Rijkers DTS et al., Thromb Haemost 1997; Supplement; 550 Abstract PS-2251) und in der US 5,051,357 beschrieben.

Diese bisher beschriebenen Verfahren zur Aktivierung des Protein C der Probe unter Verwendung des endogenen Prothrombin der Probe und von exogen zugegebenem Thrombomodulin sind dadurch gekennzeichnet, daß
1. eine Vorinkubation zur Entstehung von aktiviertem Protein C benötigt wird;
2. die Aktivierung des endogenen Thrombins zu einer vorzeitigen Entstehung eines Fibringerinnsels führt, weshalb Fibrinogen in der Probe vor der Analyse zerstört werden muß und daher,
3. nur chromogene Nachweisverfahren möglich sind;
4. bedingt durch die Inkubationszeiten und/oder Vorbehandlung der Probe insgesamt eine lange Meßdauer (größer 10 Minuten) resultiert.
Für die Analyse des Protein C System Potentials wären aber solche Methoden vorteilhaft, die eine routinemäßige Bestimmung an gängigen Koagulometem erlauben, d.h. kurze Meßzeiten (kleiner 10 Minuten) und die traditionelle Bestimmung eines Fibringerinnsels erlauben.

Eine Aufgabe der Erfindung war es daher, ein Verfahren zu finden, das es erlaubt das Protein C System Potential auch in klassischen Verfahren und mit kuren Meßzeiten zu bestimmen. Eine weitere Aufgabe war es, daß solch ein Test auch Defizite des Antithrombin III miterfaßt.

Ein Verfahren zur Bestimmung des Protein C Potentials wird von Matschiner in der US 5,525,478 beschrieben, dabei wird die Probe mit einem Kontaktphasenaktivator inkubiert und anschließend anstelle von Calciumchlorid mit einem Gemisch aus Calciumchlorid und Thrombomodulin die Gerinnung aktiviert. Matschiner führt aus, daß bei Zusatz von 1 E/ml Thrombomodulin (vom Kaninchen) die Gerinnungszeit in der APTT von 36 auf 156 s verlängert wird. Er beschreibt weiterhin davon abgeleitete Verfahren zur Bestimmung von Protein C und Protein S, indem die Probe mit Protein C bzw. Protein S Mangelplasma vor Verwendung in diesem Test gemischt wird. Eigene Versuche (siehe Beispiel 3) konnten bestätigen, daß ein Zusatz von 5 µg/ml (bezogen auf den gesamten Testansatz) Thrombomodulin (vom Kaninchen) notwendig ist um die Gerinnungszeit in der APTT von ca. 30 auf ca. 160 s zu verlängern.

Analoge Bestimmungen wurden auch mit rekombinantem Thrombomodulin durchgeführt, wie zu erwarten war, wurde eine Verlängerung der Gerinnungszeit gefunden (Ohishi et al., Thromb. Haemostas 1993; 70: 423-426). Interessanterweise ist diese Wirkung jedoch nur sehr schwach ausgeprägt (ca. 150 sec bei ca. 1 µg/ml Thrombomodulin im Plasma; ca. 1 U/ml Thrombomodulin). Diese ist allerdings nur sichtbar, wenn, wie in diesem Fall die Gerinnungszeit ohne Zusatz von Thrombomodutin sehr lange ist (450 s). Bei diesen langen Gerinnungszeiten wirken sich Verzögerungen der Thrombinentstehung überproportional auf die Gerinnselbildung aus, weshalb diese Verlängerung um 150 s nicht mit der von Matschiner beschriebenen Verlängerung bei sehr viel kürzerer Grundgerinnungszeit ohne Thrombomodulin verglichen werden kann. Diese lange Gerinnungszeit war dadurch erhalten worden, daß ein mit Calciumchlorid sehr stark verdünntes Thromboplastin-Reagenz verwendet wurde. Diese langen Gerinnungszeiten (größer 300 s) werden vom Fachmann sehr kritisch gesehen, denn die Präzision der Bestimmung ist sehr ungenau. Geringe Schwankungen an Gerinnungsfaktoren, insbesondere der Kofaktoren V und VIII führen zu überproportional starken Verlängerungen der Gerinnungszeiten. Zudem sind diese langen Meßzeiten für Routinebestimmungen unpraktikabel, da .dadurch der Probehdurchsatz in der Routine verringert wird.

Wird anstelle einer hoch verdünnten PT eine normale PT verwendet, kann der antikoagulatorische Effekt von Thrombomodulin nur durch den Einsatz sehr hoher Mengen gezeigt werden. Dies wird aus den Untersuchungen von Takahashi et al. ersichtlich (Thromb Haemostas 1995; 73: 805-811). Die Autoren setzten aus Urin angereichertes Thrombomodulin zu normalem Plasma zu und führten eine normale PT durch mit Gerinnungszeiten ohne Thrombomodulin von ca. 13 sec. Durch Zusatz von sehr hohen Konzentrationen wurde die Gerinnungszeit verlängert, doch selbst bei 1000 U/ml betrug die Verlängerung der Gerinnungszeit nur ca. 17 sec. Dies ist für eine Diskriminierung zwischen normalen Personen und Patienten mit Mangelzuständen des Protein C Systems ungenügend.

Überraschenderweise wurde gefunden, daß Thrombomodulin, von dem die Heparinähnliche Glycosilierung mittels Chondroitinase ABC entfernt wurde, in dem von Matschiner beschriebenen Verfahren keine derartig ausgeprägte Verlängerung der Gerinnungszeit aufweist.

Rekombinant hergestelltem Thrombomodulin fehlt ebenfalls die geeignete Glykosylierung um die Inaktivierung von Thrombin durch Antithrombin III zu beschleunigen (Wirkung B), d.h. es weist nur die Eigenschaft der Aktivierung von Protein C als Cofaktor für Thrombin auf (Wirkung A).

Der vorliegenden Erfinduring lag also die Aufgabe zugrunde, ein Screening-Verfahren zur Bestimmung des antikoagulatorischen Potentials zur Verfügung zu stellen. Unter antikoagulatorischem Potential wird die Eigenschaft des Plasmas verstanden auf Grund der direkten Hemmung von Thrombin und/oder der Verzögerung der Entstehung von Thrombin in einem auf der Thrombinentstehung basierenden Gerinnungstest eine Verlängerung der Gerinnungszeit zu erzeugen.

Screening-Verfahren stellen besondere Anforderungen.Da sie dazu bestimmt sind, eine hohe Anzahl Proben in kurzer Zeit und trotzdem sehr zuverlassig abzuarbeiten, sollten sie eine Meßzeit von 5 min nicht überschreiten und vorteilhafterweise als 1 Schritt-Assay durchgeführt werden können. Unter 1-Schritt-Assay wird ein Test verstanden, bei - dem zwischen den Reagenzzugaben keine Vorinkubationszeiten, nötig sind. Für das Screening größerer Personengruppen ist die Verwendung möglichst konstant Zu produzierender Reagenzien vorteilhaft, wie z.. B, die Verwendung von rekombinanten Proteinen, hier z. B. von rekombinantern Thrombomodulin. Ein Screening-Verfahren muß also auch mit einem solchen rekombinanten Thrombomodulin welchen Ursprung es auch immer hat, funktionsfähig sein. Diese Aufgabe wurde gelöst durch die in den Ansprüchen bereitgestellten Ausführungsformen.

Gegenstand der Erfindung ist ein ein Screening-Verfahren zur Bestimmung und Diagnose des antikoagulatorischen Potentials einer Probe in Anwesenheit von exogen zugesetz-tern Thrombomodulin *1 welches folgende Schritte einschließt.
*1:Humaner oder rekombinanten Ursprungs, das neben seiner Protein C aktivierenden Aktivität such die Eigenschaft zur Beschleunigung der inhibierung von Thrombin durch Antithrombin III aufweist, wobei bei recombinant hergestelltem Thrombomodulin die Thrombin Eigenschaft durch Verknuphing oder Zugabe eines Gly wiederhargestalf wird.
a) zu der Probe, bevorzugterweise einer Plasmaprobe, werden folgende Reagenzien zugegeben:
   i) exogenes Thrombomodulin humanen oder rekombinanten Ursprungs, das einen Komplex mit Thrombin bilden kann, wobei dieser Komplex das Protein C in der Probe aktivieren kann, wobei das Protein C endogenes oder exogen zugesetztes Protein C sein kann,
   ii) ein Aktivator der ohne weitere Zwischeninkubation zur Aktivierung von Prothombin zu Thrombin führt, wobei das.Prothrombin endogenes oder exogen zugesetztes Prothrombin sein kann,
   iii) Phospholipide,
   iv) Calciumionen,
   v) sowie weitere Zusatzreagenzien, die zur Optimierung von Gerinnungstesten allgemein verwendet werden,
b) die Reaktion wird durch die Zugabe des Prothrombin-Aktivator-haltigen Reagenz gestartet, und
c) die Entstehung von Thrombin wird bestimmt durch Messung der Umsatzrate eines Thrombinsubstrates, wobei diese Umsatzrate durch die Meßzeit bis zur Entstehung eines Fibringerinnsels oder durch die Umsatzrate eines markierten Thrombinsubstrats ermittelt wird.

Als Probe kann verwendet werden Vollblut aus Venen oder Kapillaren und Plasma, bevorzugterweise Citrat-Plasma.

Als Aktivatoren, die ohne weitere Zwischeninkubation zur Aktivierung von Prothrombin zu Thrombin führen, können bevorzugterweise verwendet werden: Faktor Xa oder Va oder Faktor Xa/Va-Komplexe oder dem Fachmann an sich bekannte Prothrombin-Aktivatoren aus Schlangengiften, z.B. Ecarin oder Textarin (Rosing J, Tans G, Thromb. Haemostas 1991; 65: 627-630) bzw. Faktor X Aktivatoren wie Faktor IXa, VIIIa oder Faktor IXa/VIIIa-Komplexe oder dem Fachmann an sich bekannte Faktor X und/oder Faktor V Aktivatoren aus Schlangengiften, z.B. aus Russell's Viper Venom, bevorzugterweise aber durch Zusatz eines Thromboplastin-haltigen Reagenzes, wie z.B. aus Kaninchen-Hirn, -Lunge oder aus humaner Plazenta, wie z.B. Thromborel S (Fa. Behring Diagnostics), oder rekombinanten Ursprungs, wie z.B. Innovin (Fa. Dade) oder Thromborel R (Fa. Behring Diagnostics).

Die zugesetzten Phospolipide können natürlichen oder synthetischen Ursprungs sein, bevorzugterweise aus Gewebeextrakten von Plazenta, Lunge, Hirn oder Thrombozyten humanen oder tierischen Ursprungs, bevorzugt sind auch Extrakte aus Pflanzen wie etwa Sojabohnen. Vorteilhafterweise werden die Phospholipide in einer solchen Menge zugesetzt, daß im Testansatz eine Konzentration von 0,001 % bis 1,0 % (w/v), bevorzugterweise von 0,005 % bis 0,5 %, besonders bevorzugterweise von 0,015 % bis 0,15 % erhalten wird.

Das erfindungsgemäße Verfahren kann auch verwendet werden, um selektiv Defekte spezieller Gerinnungsfaktoren zu erfassen. Dazu wird die verwendete Probe, bevorzugterweise vor ihrer Verwendung im Test, mit einer Lösung versetzt, die die Gerinnungsfaktoren enthält, die nicht im Test mit erfaßt werden sollen.

Gerinnungsfaktoren, von besonderem Interesse sind z. B. AT III, Protein S, Protein C, Faktor V und Prothrombin bzw. deren Abarten.

Zur Beseitigung von Störungen durch Heparin kann entweder in der Probe befindliches Heparin abgebaut oder neutralisiert werden, z. B. durch Heparinase oder Amine, wie Poly-Lysin, Hexadimethrin, Spermin, Spermidin oder Protaminsulfat, oder es kann ein gegenüber den zu erwartenen Heparinkonzentrationen möglichst großer Überschuß, bevorzugterweise 0,1 bis 10 U/ml Testansatz, besonders bevorzugt 0,3 - 3 U/ml, ganz besonders bevorzugt 0,7 U/ml zugegeben werden.

Aus Untersuchungen des Effektes verschiedener Thrornbomoduline wurde ge schlossen, daß die Inhibierung des Thrombins nicht nur eine von mehreren Eigenschaften des Thrombomodulins ist, sondern Voraussetzung für die antikoagulatorische Aktivität über das Protein C System. Diese Erkenntnis ist neu. Weiterhin wurde gefunden, daß die Glykosylierung des Thrombomodulins für die Beschleunigung der antikoagulatorischen Wirkung von Antithrombin III verantwortlich ist und daher wird durch daß erfindungsgemäße Verfahren neben dem Protein C System ein weiterer wichtiger antikoagulatorischer Mechanismus, das Antithrombin III selbst erfaßt. Damit wird hier zum ersten Mal ein Verfahren beschrieben, mit dem beide wichtigen Kontrollmechanismen der Gerinnung erfaßt werden.

Die Ursache für diese überraschenden Effekt ist möglicherweise weniger die mit der Hemmung des Thrombins verbundene Hemmung der Fibrinogenspaltung, sondern wahrscheinlich vielmehr die Hemmung der Faktor V-Aktivierung, diese ungebremste Aktivierung würde zu einer um den Faktor ca. 10,000 verstärkten Nachlieferung von Thrombin führen, die dann von Thrombomodulin nicht mehr so effektiv abgefangen werden kann.

Auf Grund dieser neuen Erkenntnisse ist bei einem Antithrombin III-Mangel im Gegensatz zu einem normalen Plasma mit einer verkürzten Gerinnungszeit in Gegenwart von Thrombomodulin zu rechnen, da Antithrombin III die prokoagulatorischen Aktivitäten des Thrombins im Komplex mit Thrombomodulin nicht mehr neutralisieren kann. Auch bei einem Defekt oder einer Störung des Protein C Systems kommt es im Vergleich zu einem normalen Plasma zu einer weniger ausgeprägten Verlängerung der Gerinnungszeit. Defekte in beiden Systemen wirken somit gleichsinnig. Dies konnte in Beispiel 6 erstmalig gezeigt werden.

Zur Abbildung der physiologischen Funktion des Protein C Systems und des Antithrombin III muß daher Thrombomodulin mit intakter Glykosylierung verwendet werden, d.h. das verwendete Thrombomodulin muß sowohl die Thrombin hemmende (Aktvität B) als auch die Protein C aktivierende (Aktivität A) Aktivität aufweisen. Aufgrund dieser Erkenntnis ist es möglich, die prokoagulatohsche Aktivität mittels eines Thromboplastin-haltigen Reagenz zu bestimmen, da dies den physiologisch relevanten, extrinsischen Gerinnungsweg abbildet und eine Vorinkubation der Probe zur Aktivierung der Kontaktphase entfällt (Beispiel 3).

Zur Realisierung eines auf Thromboplastin basierenden Verfahrens muß die Konzentration des Thromboplastins so gewählt werden, daß die Entstehung von Thrombin so langsam vor sich geht, daß während dieses Zeitraums genügend aktiviertes Protein C ensteht um die zur Gerinnselbifdung notwendige Entstehung von Thrombin zu verzögern. Hierzu stellt der Fachmann die Thromboplastin Konzentration in einem Reagenz so ein, daß die Gerinnungszeit eines normalen Plasmas in Abwesenheit von Thrombomodulin mindestens 20 s und höchstens 300 s beträgt, bevorzugterweise im Bereich 40 bis 150 s liegt. Dies kann z. B. dadurch erzielt werden, daß kommerzielle Thromboplastin-Reagenzien verdünnt werden. Zur Verdünnung wird bevorzugterweise eine Lösung verwendet, die die zur Gerinnungsaktivität notwendigen Calciumionen enthält.

Weiterhin sollten auch Phospholipide in geeigneter Menge (0,001 bis 1 % w/v) und Art (vorzugsweise aus Gewebeextrakten, wie Thrombozyten, Lunge, Plazenta oder Hirn oder aus pflanzlichen Quellen) bei der Verdünnung des Reagenzes substituiert werden. Diese Quellen enthalten meistens genügend hone Anteile von Phosphatidylethanolamin, einem Phospholipid, das für die Aktivität von aktiviertem Protein C wichtig ist. Doch kann dies nach Bedarf auch zudosiert werden um die Wirkung A des Thrombomodulins und des Protein C Systems zu stimulieren.

Zur Ermittlung der optimalen Kombination von Thromboplastin-Konzentration und Thrombomodulin-Konzentration wird eine Kurvenschar erstellt bei der die Gerinnungszeit mit und ohne einer bestimmten Konzentration von Thrombomodulin in Abhängigkeit von der Verdünnung des Thromboplastins ermittelt wird und dies bei verschiedenen Thrombomodulin-Konzentrationen wiederholt (siehe Beispiel 4).

Bevorzugterweise werden ThromuumoduHn-Konzentrationen zwischen 0,5 und 50 µg/ml bezogen auf das Endvolumen des Testansatzes eingesetzt, besonders bevorzugt Konzentrationen zwischen 1 und 10 µg/ml. Aus dieser Kurvenschar erweisen sich folgende Kombinationen als geeignet, die in Gegenwart von Thrombomodulin Gerinnungszeiten mit einem Normalplasma kleiner 300 sec, besonders bevorzugt kleiner 150 s, aufweisen und bei denen die Differenz zur Gerinnungszeit ohne Thrombomodulin mindestens 50 %, bevorzugterweise 100 - 300 % dieser Gerinnungszeit ohne Thrombomodulin beträgt.

Das Thromboplastin kann natürlichen Quellen, wie Plazenta, Lunge oder Hirn humanen Ursprungs entstammen, ats auch mit gentechnischen Mitteln erzeugt worden sein.

Das Thrombomodulin wird bevorzugterweise aus natürlichen Quellen, wie Plazenta, Lunge oder Hirn humanen Ursprungs mit dem Fachmann an sich bekannten Verfahren isoliert. Es ist dadurch gekennzeichnet, daß die Thrombomodulin-haltigen Fraktionen neben der Protein C Aktivierung auch eine durch Antithrombin III-verstärkte anti-Thrombin Wirkung aufweisen.

Es ist auch bekannt, Thrombomodulin rekombinant herzustellen. Dem unglykolisierten rekombinanten Thrombomodulin muß die Aktivität B durch biochemische oder chemische Kopplung mit einem Heparinsulfat posttranslational hinzugefügt werden. Dies kann auch durch eine Expression in glykolisierenden Zellen, z. B. humanen Ursprungs, erfolgen. Überraschenderweise wurde gefunden, daß die Zugabe von nicht an Thrombomodulin gebundenem Heparinsulfat bereits die nötige Wirkung erzielt (Beispiel 7).

Zur Vermeidung einer frühzeitiger Gerinnselbildung können dem Reagenz bekannte Aggregationsinhibitoren, wie Fibrinspaltprodukte, erhalten durch Spaltung von Fibrinogen durch Cyanbromid, Plasmin, Elastase oder andere bekannte Enzyme, etwa aus Schlangengiften (siehe beispielsweise Markland FS Jr., Thromb. Haemostas. 1991; 65: 438-443), oder synthetische Peptide, die die Sequenz RGD aufweisen, wie beispielsweise in EP- A-0 456 152 beschrieben, zugesetzt werden.

Um eine Oxidation des Thrombomodutins oder der Phospholipide im Reagenz auszuschließen können als Oxidationsschutz dem Fachmann an sich bekannte Substanzen wie Kaliumhexacyanoferrat, Vitamin C, Glutathion, Harnsäure. Hydrochinon. Tocopherole, Butyt-Hydroxy-Toluol (BHT), Butyl-Hydroxy-Anilin, Ubichinon oder Enyzme wie Superoxiddismutase und Katalase verwendet werden.

Auf Basis dieses neuen Verfahrens können durch Zusatz zu den Reagenzien oder Proben einzelne Teile der antikoagulatorischen Systems sichtbar gemacht werden. So kann beispielsweise Antithrombin III zugesetzt werden um nur Störungen des Protein C Systems zu erkennen. Umgekehrt kann die Probe beispielsweise mit einem Antithrombin III Mangelplasma gemischt werden, um Störungen des Protein C Systems auszublenden. Weiterhin können Plasmen, die einen oder mehrere Faktoren des Protein C Systems nicht enthalten, sei da es sich um natürliche, etwa congenitale Mangelplasmen handelt oder da diese Faktoren technisch, etwa immunadsorptiv entfernt wurden, verwendet werden um das Probenplasma damit zu versetzen, so daß nur Störungen von Faktoren deutlich werden, die im zum Mischen verwendeten Plasma fehlen. Zur Neutralisierung von Anti-Phospholipid-Antikörpern, beispielsweise Lupus Antikoagulantien, können auch Phospholipide, etwa von Thrombozyten, zu Reagenzien oder direkt zur Probe und/oder Mangelplasmen zugesetzt werden.

Die Beobachtung, daß für eine deutliche Verzögerung der Gerinnungsaktivität über das Protein C System die antikoagulatorische Wirkung (Aktivität B) des Thrombomodulins notwendig ist führt zu einer neuen Interpretation hinsichtlich der biologischen Bedeutung des Kohlenhydratanteils und der diagnostischen Anwendung. Bisher ist die eigentliche biologische Bedeutung des Kohlenhydratanteils von Glykoproteinen unbekannt. In der Regel wird nur ein Einfluß auf die Halbwertszeit im Kreislauf diskutiert (Paulson JC, TIBS 1989; 14: 272-275). Nun ist bekannt, daß Diabetiker eine höhere Inzidenz von Thrombosen, insbesondere im arteriellen Gefäßsystem aufweisen. Da auf Grund der hier vorgestellten Schlußfolgerungen die antikoagulatorische Wirkung auf Thrombin die Voraussetzung für die antikoagulatorische Protein C Aktivität ist und andererseits bei Diabetikern eine Störung der korrekten Synthese des Kohlenhydratanteils auftreten kann, wird vermutet, daß der Verlust der anti-Thrombin Aktivität des Thrombomodulins bei Diabetikern einen wichtigen Teil des Pathomechanismus spielt, der zu einem erhöhten Thromboserisiko führt.

Dies bedeutet, daß der Nachweis der Glykosilierung bzw. der Aktivität B (Anti-Thrombin Wirkung) des Thrombomodulins im Verhältnis zur Aktivität A (Protein C Aktivierung) ein wichtiger diagnostischer Marker für das antikoagulatorische Protein C Potential auf der Gefäßoberfläche darstellt und damit zur Risikoabschätzung einer Thrombosegefährdung insbesondere für arterielle Thrombosen genutzt werden kann. Bevorzugterweise findet diese Analyse bei Diabetikern oder Personen mit gestörtem Methionin-Stoffwechsel (Hyperhomocysteinaemie) statt um den Fortschritt der Endothelschädigung zu erfassen. Auch für andere Erkrankungen, die mit einer Störung des Metabolismus des Endothels assoziiert sind, wie beispielsweise Tumore, Atherosklerose, Autoimmunerkrankungen oder andere entzündliche Erkrankungen, kann diese Analyse einen wichtigen prognostischen oder therapeutisch sinnvollen Hinweis geben.

Der Nachweis des Verhältnisses der beiden Aktivitäten kann mit natürlicherweise im Plasma vorkommenden Spaltprodukten des Thrombomodulins erfolgen, als auch durch Analyse von natürlichem Gewebe von Patienten. Die Bestimmung der beiden Aktivitäten erfolgt in chromogenen Testen wie sie beispielsweise bei Preissner et al. beschrieben sind (J. Biol.Chem. 1990; 265: 4915-4922; siehe auch Beispiel 1). Bevorzugterweise wird vor der Bestimmung das Thrombomodulin von der restlichen Matrix (etwa Plasmabestandteile) abgetrennt. Hierzu ist ein Testkit bestehend aus einer mit Antikörpern gegen Thrombomodulin beschichteten Festphase, beispielsweise einer Mikrotiterplatte, Teststreifen oder Testmodul, geeignet. In einem ersten Inkubationsschritt wird das Thrombomodulin an die Festphase gebunden und anschließend durch Waschen störende Matrix abgetrennt. Anschließend werden die Anteile beider Aktivitäten in getrennten Testansätzen in chromogenen Verfahren bestimmt.

Weiterhin kann der Glykosylierungsgrad des aus Blut oder Gewebe von Patienten isolierten Thrombomodulins über dem Fachmann an sich bekannte Verfahren direkt bestimmt werden. Die Ergebnisse, etwa das Verhältnis aus Aktivität A zu Aktivität B oder vice versa, dienen als Maß des Schweregrads der Syhthesestörung bzw. als Indikator eines erhöhten Thromboserisikos.

### Beispiele

Die folgenden Beispiele unter Verwendung von Thrombomodulin vom Kaninchen sollen die Erfindung nur erläutern. So weit nicht anders vermerkt wurden Reagenzien und Geräte der Fa. Behring Diagnostics GmbH verwendet:

### Beispiel 1

### Chromogene Bestimmung der Thrombomodulin Aktivität A (Protein C Aktivierung)

Die chromogene Bestimmung der Thrombomodulin Aktivität erfolgt in Anlehnung an Salem et al., Journal of Biological Chemistry, Vol 259, No. 19, S. 12246-12251 (1984). Die Testdurchführung erfolgte an einem Behring Coagulation Timer (Behring Diagnostics):
50 µl Probe wurden mit 50 µl Reagenz 1, beinhaltend 50 µg/ml Protein C, 6 µg/ml Thrombin in 50 mM Tris-HCl, 200 mM NaCl, 5 mM MnCl₂, 1 % Rinderserumalbumin, pH 7,3 gemischt und 5 Minuten inkubiert. Während dieser Zeit aktiviert Thrombin in Zusammenwirkung mit dem in der Probe enthaltenen Thrombomodulin Protein C zu aktiviertem Protein C. Diese Aktivierung wird durch Zugabe eines Inhibitor-Cocktails (50 E/ml Antithrombin III, 5 anti-Thrombin Einheiten/ml Hirudin, 1 E/ml unfraktioniertes Heparin in 50 mM Tris-HCl, 100 mM NaCl, 5 mM EDTA, pH 7,4.) unterbrochen und nach 30 Sekunden 50 µl eines chromogenen Protein C Substrats (aus Berichrom Protein C; Behring Diagnostics) zugegeben. Die Farbentwicklung wird über 30 Sekunden bei 405 nm verfolgt und daraus die Änderung der Exktinktion pro Minute (Delta-E/min) berechnet. Diese Änderung ist der Menge an Thrombomodulin in der Probe proportional. Die in Tabelle 1 aufgeführten Werte wurden mit Kaninchen-Thrombomodulin (Fa. American Diagnostica; 1000 E/mg Protein) erhalten.

**Tabelle 1. Bestimmung der Thrombomodulin-Aktivität im chromogenen Test. (TM deglyc = mit Chondroitinase-behandeltes Thrombomodulin; siehe Beispiel 2)**

| Thrombomodulin (µg/ml) | Delta-E/min * 10⁻³ |
|---|---|
| 0 | 20,0 |
| 0,2 | 53,9 |
| 0,4 | 81,0 |
| 0,6 | 106,0 |
| 0,8 | 155,1 |
| 1,0 | 169,0 |
| 1,5 | 210,7 |
| 2,0 | 247,3 |

| TM decgly. (µg/ml) | Delta-E/min * 10⁻³ |
|---|---|
| 0,5 | 113,4 |

### Beispiel 2

### Entfernung des Kohlenhydrat-Anteils des Thrombomodulins

Zur Entfernung des Heparin-Anteils des Thrombomodulins wurden 1 ml mit 30 µg/ml Kaninchen-Thrombomodulin (Fa. American Diagnostica) in 50 mM Tris-HCl pH 8,0 mit 30 µl einer Chondroitinase ABC-Lösung (10 E/ml; Fa. Sigma) versetzt und bei +37 °C über Nacht inkubiert.

Das behandelte Thrombomodulin wurde auf 0,5 µg/ml in Reagenz-Puffer 1 aus Beispiel 1 verdünnt und getestet. Das vorbehandelte Thrombomodulin zeigte bei 0,5 µg/ml eine Aktivität, die 0,6 µg/ml des unbehandelten Thrombomodulins entspricht.

Die Protein C aktivierende Aktivität war somit nicht reduziert, sondern vielmehr leicht erhöht.

### Beispiel 3

### Verlängerung der APTT durch Zusatz von Thrombomodulin

Wie bei Matschiner (US 5,525,478) angegeben, wurden 50 µl eines Poolplasmas von normalen Spendern mit 50 µl eines APTT-Reagenzes (Actin; Fa. Dade) versetzt und 120 sec bei +37 °C zur Aktivierung der Kontaktphase inkubiert. Anstelle des sonst bei einer APTT zugesetzten Calciumchlorids wurden 50 µl Thrombomodulin (vom Kaninchen; 15 µg/ml in physiologischer Kochsalzlösung) zugegeben und erst anschließend mit Calciumchlorid (25 mmol/l) die Gerinnung ausgelöst. Die Ergebnisse sind in Tabelle 2 aufgeführt. Sie zeigen zunächst, daß in Anwesenheit von Thrombomodulin die Gerinnungszeiten gegenüber einer normalen Probe verlängert werden. Dies entspricht den Angaben von Matschiner.

Allerdings tritt eine solche, wenngleich geringere Verlängerung auch bei Protein C Mangelplasma auf. In diesem Fall wird vermutet, daß dies nicht auf die Inaktivierung der prokoagulatorischen Kofaktoren VIIIa und Va (Aktivität A) zurückgeführt werden kann, sondern auf die Hemmwirkung (Aktivität B) des Thrombomodulin gegenüber Thrombin. Dies wird deutlich, wenn wie in diesem Beispiel Thrombomodulin eingesetzt wird, dessen Glykosylierung entfernt wurde. Überraschenderweise entfällt nun nicht nur die durch Aktivität B bedingte Gerinnungsverlängerung, sondern auch (ca. 20 sec; = Differenz aus Protein C Mangelplasma mit und ohne Thrombomodulin) nahezu auch völlig die durch Aktivität A bedingte Gerinnungsverlängerung.

Dies wurde bisher nicht gezeigt. Daraus ergibt sich der Schluß, daß Aktivität B eine Voraussetzung für Aktivität A unter physiologischen Bedingungen (Fibrinbildung) ist und somit im Gerinnungstest nur Thrombomodulin mit intakter. Glykosylierung eingesetzt werden kann.

**Tabelle 2. Einfluß von Thrombomodulin (3,75 µg/ml im Testansatz) auf die APTT von Normalplasma und Protein C-Mangelplasma. Angegeben sind die Gerinnungszeiten in sec. TM = Thrombomodulin. Intakt = natürliches, glykosyliertes Thrombomodulin; deglyc = nach Behandlung mit Chondroitinase ABC (siehe Beispiel 2).**

| TM-Zusatz | Normalplasma | Protein C Mangelplasma |
|---|---|---|
| ohne | 28,2 | 32,7 |
| TM. intakt | 166,4 | 63,2 |
| TM deglyc | 40,4 | 44,6 |

### Beispiel 4

### Verlängerung der PT eines Normalplasmas durch Thrombomodulin in Abhängigkeit von der Konzentration an Thromboplastin und Thrombomodulin

Im Verfahren muß zu einer gegebenen Konzentration von Thrombomodulin eine geeignete Konzentration eines Thromboplastin-haltigen PT-Reagenzes gesucht werden, um eine für einen Screening Test für das Protein C System geeignete Verlängerung der Gerinnungszeit (20 - 300 sec) zu erzielen.

Hierzu wurde 1 Teil Probe mit 1 Teil eines Thrombomodulin-haltigen Reagenzes (Thrombomodulin vom Kaninchen in 50 mM Tris-HCl, ohne bzw. mit 0,025% Phospholipid aus Sojabohnen, pH 7,4) versetzt und die Gerinnungsreaktion mit einem PT-Reagenz in verschiedenen Verdünnungen (hier beispielsweise Thromborel S, Behring Diagnostics; Verdünnung mit 25 mM Calciumchlorid-Lösung) ausgelöst.

Zum Vergleich wurde die Gerinnungszeit ohne Zusatz von Thrombomodulin bestimmt.

Das in Tabelle 3 aufgeführte Beispiel zeigt, daß bei niedriger Konzentration von Thrombomodulin gegenüber einer PT ohne Thrombomodulin mit steigender Verdünnung des Thromboplastins die Gerinnungszeiten zunächst gleich bleiben und erst mit hoher Verdünnung (hier: 1:1000) eine Differenzierung erzielt wird. Wird hingegen die Thrombomodulin Konzentration höher gewählt, so ist eine Differenzierung bereits bei einer niedrigeren Verdünnung erkennbar. In diesem Versuch würde eine optimale Kombination bei einer 1:100 Verdünnung des Thromboplastins in Gegenwart von 1,7 µg/ml Thrombomodulin (im Testansatz) gewählt werden (siehe auch Beispiel 5), da die Differenzen bei niedrigeren Verdünnungen von Thromboplastin zu niedrig sind. Bei der 1:100 Verdünnung ist die Differenz mit 1,4 µg/ml etwas niedrig, mit 2,0 µg/ml etwas hoch. Damit benötigt dieses Verfahren deutlich weniger Thrombomodulin im Testansatz als das Verfahren nach Matschiner und ist diesem damit überlegen.

Ohne Substitution mit Phospholipiden werden bei höherer Verdünnung längere Gerinnungszeiten ohne und mit TM erhalten als bei Substitution mit Phospholipiden (siehe Tabelle 4). Die Differenzierung zwischen dem Kontrolltest ohne Thrombomodulin und dem eigentlichen Screeningtest mit. Thrombomodulin ist ohne Substitution mit Phospholipiden etwas schlechter. Bei der Substitution mit Phospholipiden muß beachtet werden, daß die Empfindlichkeit für Lupus Antikoagulantien damit im Verfahren sinkt.

**Tabelle 3 Einfluß der Thromboplastin-Konzentration auf die PT eines Normalplasmas in An- und Abwesenheit verschiedener Konzentration Thrombomodulin bei konstanter Phospholipid-Konzentration. Angegeben sind Gerinnungszeiten in sec sowie die Differenzen zur Gerinnungszeit ohne Thrombomodulin im Ansatz. TM = Thrombomodulin; PL = Phospholipide; Diff. = Differenz zwischen Gerinnungszeit mit und ohne TM;**

| Verdünnung | 0 µg/ml | 0,7 µg/ml | Diff. | 1,4 µg/ml | Diff. | 2,0 µg/ml | Diff. |
|---|---|---|---|---|---|---|---|
| 1:10 | 22,9 | 24,0 | 1,1 | 24,6 | 1,7 | 25,7 | 2,8 |
| 1:30 | 31,5 | 35,2 | 3,7 | 35,6 | 4,1 | 41,9 | 10,4 |
| 1:100 | 44,7 | 53,9 | 9,2 | 83,9 | 39,2 | 231,8 | 187,1 |
| 1:300 | 62,7 | 101 | 38,3 | >300 | | >300 | |
| 1:1000 | 103,9 | >300 | | >300 | | >300 | |

**Tabelle 4 Einfluß der Thromboplastin-Konzentration auf die PT eines Normalplasmas in An- und Abwesenheit verschiedener Konzentration an Phospholipiden bei konstanter Thrombomodulin-Konzentration (0,7 µg/ml). Angegeben sind Gerinnungszeiten in sec sowie die Differenz zwischen der Gerinnungszeit mit und ohne Thrombomodulin im Ansatz. TM = Thrombomodulin; PL = Phospholipide**

| Verdünnung | ohne TM mit PL | mit TM mit PL | Differenz mit PL | ohne TM ohne PL | mit TM ohne PL | Differenz ohne PL |
|---|---|---|---|---|---|---|
| 1:10 | 22,9 | 24,0 | 1,1 | 22,5 | 23,7 | 1,2 |
| 1:30 | 31,5 | 35,2 | 3,7 | 33,5 | 37,8 | 4,3 |
| 1:100 | 44,7 | 53,9 | 9,2 | 54,8 | 65,4 | 10,6 |
| 1:300 | 62,7 | 101 | 38,3 | 84,9 | 109,6 | 24,7 |
| 1:1000 | 103,9 | >300 | >200 | 154,2 | 281,9 | 127,7 |

### Beispiel 5

### Verhalten von Plasmen mit Defekten im Antikoagulatorischen System im Verfahren

Aus nach Beispiel 4 ermittelten Kurvenscharen wurde eine optimale Kombination von Aktivatorreagenz (hier: Thromboplastin) und Thrombomodulin ermittelt. In Tabelle 5 ist die Reaktion von verschiedenen Plasmen mit Defekten im Protein C System für eine solche Kombination gezeigt. Wie in Beispiel 4 wurde Thromborel S 1:100 mit Calciumchlorid verdünnt. Das Thrombomodulin Reagenz enthielt 5,0 µg/ml Thrombomodulin (entspricht 1,7 µg/ml im Testansatz) sowie Phospholipide (0,025%). Folgende Plasmen wurden getestet: ein normales Poolplasma, ein Protein C Mangelplasma, ein Protein S Mangelplasma, sowie ein Faktor V Leiden Plasma. Die Ergebnisse in. Tabelle 5 belegen, daß unter diesen Bedingungen die Gerinnungszeiten der pathologischen Proben gegenüber dem Normalplasma in Anwesenheit von Thrombomodulin verkürzt waren.

Dies zeigt, daß ein Test basierend auf einer verdünnten PT in Anwesenheit von Thrombomodulin Störungen des Protein C Systems durch eine weniger ausgeprägte Verlängerung der Gerinnungszeit anzeigt.

**Tabelle 5 Gerinnungszeiten (in sec) von verschiedenen Plasmen mit Defekten im Protein C System im Vergleich zu einem Normalplasmapool im Verfahren. TM = Thrombomodulin**

| Probe | ohne TM | mit TM | Differenz (mit-ohne) TM | Quotient (mit/ohne) TM |
|---|---|---|---|---|
| Normalplasma | 34,3 | 125,6 | 91,3 | 3,7 |
| Protein C MP | 37,4 | 62,9 | 25,5 | 1,7 |
| Protein S MP | 41,3 | 68,4 | 27,1 | 1,7 |
| Faktor V Leiden (heterozygoter Defekt) | 39,8 | 103,6 | 63,8 | 2.6 |

### Beispiel 6

### Verhalten eines Plasmas Antithrombin III Mangel im Verfahren

Ein Plasma mit kongenitalem Antithrombin III Defekt (< 0.01 U/ml AT III; Fa. Milan Analytica AG, Schweiz) aber sonstigen Gerinnungsfaktoren im Normbereich wurde wie in Beispiel 5 beschrieben im Verfahren eingesetzt. Das Thrombomodulin Reagenz enthielt abweichend von Beispiel 5 7,0 µg/ml Thrombomodulin (entspricht 2,3 µg/ml im Testansatz) üm wie in Beispiel 4 gezeigt eine optimale Reaktion zu erzielen. Zum Vergleich wurde das gleiche normale Poolplasma wie in Beispiel 5 mitgeführt.

Die Ergebnisse in Tabelle 6 belegen, daß die Gerinnungszeit des Antithrombin-defizienten Plasmas gegenüber dem Normalplasma in Anwesenheit von Thrombomodulin verkürzt war. Diese Verkürzung deutet auf eine gegenüber einem Normalplasma unvollständiges Antikoagulationspotentiai hin. Somit erlaubt das Verfahren erstmalig sowohl die Detektion von Defekten in beiden wichtigen antikoagualatorischen Systemen: dem Protein C System als auch des Antithrombin III.

**Tabelle 6 Gerinnungszeiten (in sec) eines Plasmas mit Antithrombin III Mangel (<0.001 U/ml) im Vergleich zu einem Normalplasmapool im Verfahren. Angegeben sind die Gerinnungszeiten mit und ohne Thrombomodulin, sowie die Differenz und der Quotient aus beiden Gerinnungszeiten. TM = Thrombomodulin**

| Probe | ohne TM | mit TM | Differenz (mit - ohne) TM | Quotient (mit/ohne) TM |
|---|---|---|---|---|
| Normalplasma | 37,4 | 128,5 | 91,1 | 3,4 |
| Antithrombin III MP | 42,7 | 79,9 | 37,2 | 1,9 |

### Beispiel 7

### Ersatz der natürlichen Glykosylierung durch Zusatz eines Heparinsulfats zu einem deglykosyliertem Thrombomodulin.

Wie in Beispiel 2 beschrieben wurde das Thrombomodulin vom Kaninchen durch Verwendung von Chöndroitinase deglykosyliert (TM deglyc).

Wie in Beispiel 4 wurde Thromborel S 1:100 mit 25 mmol/l. Calciumchlorid verdünnt. Das Thrombomodulin Reagenz enthielt 10 µg/ml Thrombomodulin (entspricht 3,3 . µg/ml im Testansatz) sowie Phospholipide von Sojabohnen (0,05%). Auf Grund der Deglykosylierung wird im Verfahren eine nur sehr geringe Verlängerung der erreicht, wie dies in Tabelle 7 für einen normalen Plasmapool aufgezeigt ist. Bei Zusatz von Heparin zum Thrombomodulin-haltigen Reagenz (1 U/ml; entspricht 0,33 U/ml im Testansatz; Liquemin, Fa. Hoffmann-LaRoche, Schweiz) wird durch die Inhibierung der prokoagulatorischen Reaktion auch die Gerinnungszeit in Abwesenheit von Thrombomodulin verlängert, in Anwesenheit von Thrombomodulin hingegen ist die Gerinnungszeit um ein Vielfaches verlängert. Dies ist auf die durch den Zusatz von Heparin restaurierte antikoagulatorische Eigenschaft des Thrombomodulins zurückzuführen, denn mit einem Plasma mit einem Defekt im Protein C System (heterozygotes Faktor V Leiden Defekt Plasma) ist die Differenz bzw. Ratio zwischen diesen beiden Gerinnungszeiten deutlich schwächer ausgeprägt.

Das erfindungsgemäße Verfahren kann daher auch mit nicht glykosyliertem Thrombomodulin durchgeführt werden, beispielsweise rekombinant hergestelltem Thrombomodutin, in dem Heparin dem Testansatz zugeführt wird.

**Tabelle 7 Gennnungszeiteh (in sec) von Normalplasma und einem Plasma mit einem Defekt im Protein C System (heterozygoter Faktor V Leiden Defekt) bei Verwendung von glykosyliertem Thrombomodulin (gly) oder deglykosyliertem Thrombomodulin (degly) ohne (-) bzw. mit Zusatz (hep) von 1 E/ml Heparin im Verfahren. Angegeben sind die Gerinnungszeiten mit und ohne Thrombomodulin, sowie die Differenz-und der Quotient aus beiden Gerinnungszeiten. TM = Thrombomodulin**

| Probe | TM/Heparin | ohne TM | mit TM | Differenz | Quotient |
|---|---|---|---|---|---|
| Normalplasma | gly | 32,3 | 106,8 | 74,5 | 3,3 |
| | degly / - | 35,0 | 45,7 | 10,7 | 1,3 |
| | degly / hep | 65,9 | 189,5 | 123,6 | 2,9 |
| Faktor V Leiden | degly / hep | 61,9 | 99,3 | 37,4 | 1,6 |

## Patentansprüche

1. Screening-Verfahren zur Bestimmung des antikoagulatorischen Potentials einer Probe in Anwesenheit von exogen zugesetztem Thrombomodulin humanen oder rekombinanten Ursprungs, das neben seiner Protein C aktivierenden Aktivität auch die Eigenschaft zur Beschleunigung der Inhibierung von Thrombin durch Antithrombin III aufweist, wobei bei rekombinant hergestelltem Thrombomodulin die Thrombin inaktivierende Eigenschaft durch Verknüpfung oder Zugabe eines Glykosaminoglycans wiederhergestellt wird, welches folgende Schritte einschließt:
a) zu der Probe, bevorzugterweise einer Plasmaprobe, werden folgende Reagenzien zugegeben:
i) exogenes Thrombomodulin humanen oder rekombinanten Ursprungs, das einen Komplex mit Thrombin bilden kann, wobei dieser Komplex das Protein C in der Probe aktivieren kann, wobei das Protein C endogenes oder exogen zugesetztes Protein C sein kann,
ii) ein Aktivator der ohne weitere Zwischeninkubation zur Aktivierung von Prothrombin zu Thrombin führt, wobei das Prothrombin endogenes oder exogen zugesetztes Prothrombin sein kann,
iii) Phospholipide,
iv) Calciumionen,
v) sowie weitere Zusatzreagenzien, die zur Optimierung von Gerinnungstesten allgemein verwendet werden.
b) die Reaktion wird durch die Zugabe des Pruthrombin-Aktivator-haltigen Reagenz gestartet, und
c) die Entstehung von Thrombin wird bestimmt durch Messung der Umsatzrate eines Thrombinsubstrates, wobei diese Umsatzrate durch die Meßzeit bis zur Entstehung eines Fibringerinnsels oder durch die Umsatzrate eines markierten Thrombinsubstrats ermittelt wird.

2. Verfahren nach Anspruch 1, wobei die gemessene Umsatzrate in Relation gesetzt wird zu der Umsatzrate in einem Testansatz zur Bestimmung der Gerinnungszeit, in dem kein aktiviertes Protein C entsteht oder zugesetzt wird.

3. Verfahren nach Anspruch 2, wobei die gemessene Umsatzrate in Relation gesetzt wird zu der Umsatzrate in einem Testansatz analog zu dem Verfahren nach Anspruch 1, In dem aber kein Thrombomodulin zugesetzt wird.

4. Verfahren nach mindestens einem der Ansprüche 1 bis 3, **dadurch gekennzeichnet, daß** die Konzentration des Aktivators in Schritt a) ii) so gewählt wird, daß die Gerinnungszeit eines normalen Plasmas in Abwesenheit von Thrombomodulin mindestens 20 s und höchstens 300 s, bevorzugterweise 30 bis 150 s beträgt.

5. Verfahren nach mindestens einem der Ansprüche 1 bis 4, **dadurch gekennzeichnet, daß** dem Fachmann an sich bekannte Thromboplastin-Reagenzien als Aktivatoren in Schritt a) ii) verwendet werden, die natürlichen humanen oder tierischen Ursprungs, wie aus Plazenta, Lunge oder Hirn sind oder mit gentechnischen Mitteln erzeugt werden.

6. Verfahren nach Anspruch 1 **dadurch gekennzeichnet, daß** das Thrombomodulin getrennt vom Aktivator-haltigen Reagenz in einem gesonderten Reagenz der Probe zugesetzt wird.

7. Verfahren nach Anspruch 1, **dadurch gekennzeichnet, daß** das verwendete Thrombomodulin humanen Ursprungs ist.

8. Verfahren nach Anspruch 7 **dadurch gekennzeichnet, daß** bei rekombinant hergestelltem Thrombomodulin die Thrombin inaktivierende Eigenschaft durch Verknüpfung mit Heparinsulfat wiederhergestellt wird.

9. Verfahren nach Anspruch 7 **dadurch gekennzeichnet, daß** bei rekombinant hergestelltem Thrombomodulin die Thrombin inaktivierende Eigenschaft durch Zugabe von Heparinsulfat wiederhergestellt wird.

10. Verfahren nach mindestens einem der Ansprüche 1 bis 8 **dadurch gekennzeichnet, daß** die Verknüpfung rekombinant oder synthetisch erfolgt.

11. Verfahren nach Anspruch 1, **dadurch gekennzeichnet, daß** die Menge an Thrombomodulin im Reagenz so gewählt wird, daß in Gegenwart von Thrombomodulin die Gerinnungszeiten mit einem Normalplasma kleiner 300 sec, besonders bevorzugt kleiner 150 sec, sind und daß die Differenz zur Gerinnungszeit ohne Thrombomodulin mindestens 40 %, bevorzugterweise 100 bis 300 % dieser Gerinnungszeit ohne Thrombomodulin beträgt.

12. Verfahren nach mindestens einem der Ansprüche 1 bis 11, **dadurch gekennzeichnet, daß** die Thrombomodulin-Konzentration 0,5 bis 50 µg/ml, bevorzugterweise 1 bis 10 µg/ml bezogen auf das Endvolumen des Testansatzes beträgt.

13. Verfahren nach mindestens einem der Ansprüche 1 bis 12, **dadurch gekennzeichnet, daß** zur Verzögerung der Gerinnselbildung bekannte Aggregationsinhibitoren im Testverfahren zugesetzt werden.

14. Verfahren nach einem der Ansprüche 1 bis 13, **dadurch gekennzeichnet, daß** gereinigte Gerinnungsfaktoren, die nicht an der Funktion des Protein C Systems oder des Antithrombin III beteiligt sind durch Zusatz zum Reagenz oder Reagenzien substituiert werden.

15. Verfahren nach Anspruch 14, **dadurch gekennzeichnet, daß** Fibrinogen, Faktor VII, Faktor IX, Faktor X und/oder Prothrombin (Faktor II) zum Reagenz oder Reagenzien gegeben werden.

16. Verfahren nach Anspruch 14, **dadurch gekennzeichnet, daß** Antithrombin III zugesetzt wird, um nur Störungen des Protein C Systems zur erkennen.

17. Verfahren nach Anspruch 1, **dadurch gekennzeichnet, daß** zur selektiven Erfassung einzelner oder mehrerer Störungen die Plasmaprobe vor Verwendung im Verfahren mit einer Lösung versetzt wird, die Gerinnungsfaktoren enthält, die nicht im Test mit erfaßt werden sollen.

18. Verfahren nach Anspruch 17, **dadurch gekennzeichnet, daß** zur selektiven Diagnose des Defekts oder Mangels eines Proteins die Probe vor Verwendung im Verfahren mit einem Plasma, das weniger als 5 % dieses Proteins enthält, im Verhältnis 1:2 bis 1:20, bevorzugterweise 1:3 bis 1:5, besonders bevorzugterweise 1:4 vorverdünnt wird.

19. Verfahren nach Anspruch 17, **dadurch gekennzeichnet, daß** zur selektiven Diagnose eines Defekts oder Mangels mehrerer Proteine die Probe vor Verwendung im Verfahren mit einem Plasma, das weniger als 5 % dieser Proteine enthält, im Verhältnis 1:2 bis 1:20, bevorzugterweise 1:3 bis 1:5, besonders bevorzugterweise 1:4 vorverdünnt wird.

20. Verfahren nach Anspruch 17, **dadurch gekennzeichnet daß** zur selektiven Diagnose von Anti-Phospholipid-Antikörpern die Probe vor Verwendung im Verfahren mit einer wässrigen Lösung, die Phospholipide und/oder Thrombozyten in einer Konzentration von 0,01 bis 1 % (w/v) enthält, im Verhältnis 1:2 bis 1:20, bevorzugterweise 1:3 bis 1:5, besonders bevorzugertweise 1:4 vorverdünnt wird.

21. Verfahren nach Anspruch 17, **dadurch gekennzeichnet, daß** zur Bestimmung der antikoagulatorischen Aktivität des Antithrombin III die Probe mit einem Antithrombin III Mangelplasma in einem Verhältnis 1:2 bis 1:10, bevorzugterweise 1:4, vorverdünnt wird.

22. Verfahren nach Anspruch 1, **dadurch gekennzeichnet, daß** die Substratumsatzrate der Probe in Anwesenheit von Thrombomodulin und in Abwesenheit von Thrombomodulin bestimmt wird und diese Differenz oder der Quotient aus beiden in Bezug gesetzt wird zu der Differenz oder dem Quotienten der mit einem normalen Plasma oder Plasmapool erhalten wird.

23. Verwendung des Verfahrens nach Anspruch 1 zur Erkennung von Patienten die ein erhöhtes Thromboserisiko haben.

24. Verwendung des Verfahrens nach Anspruch 1 zur Überwachung einer Antikoagulations-Therapie.

25. Verfahren zum Nachweis und der Quantifizierung der Glykosilierung des Thrombomodulins eines Patienten durch Bestimmung des Verhältnisses der Protein C aktivierenden Aktivität und der die Beschleunigung der inhibierung von Thrombin durch Antithrombin III bewirkenden Aktivität des Thrombomodulins einer Patientenprobe.

26. Verfahren nach Anspruch 25, **dadurch gekennzeichnet, daß** die Bestimmung beider Aktivitäten des Thrombomodulins in der Probe direkt erfolgt.

27. Verfahren nach Anspruch 25, **dadurch gekennzeichnet, daß** das endogene Thrombomodulin vor der Bestimmung beider Aktivitäten aus der Probe isoliert wird.

28. Ein Testkit zur Verwendung in einem Verfahren nach Anspruch 25, beinhaltend
a) einen Teststreifen beschichtet mit Antikörpern gegen Thrombomodulin, der mit der Probe in Kontakt gebracht wird,
b) eine Waschlösung, in der der inkubierte Teststreifen gewaschen wird,
c) Reagenzien für die Bestimmung der Protein C Aktivierung
d) Reagenzien für die Bestimmung der Thrombin Inaktivierung.

29. Testkit nach Anspruch 28 enthaltend Reagenzien für die Bestimmung der Protein C Aktivierung, wobei ein Reagenz Thrombin, Protein C und Calciumchlorid enthält, in dem der Teststreifen zunächst zur Aktivierung von Protein C inkubiert wird, und ein zweites Reagenz Antithrombin III, Heparin und/oder Hirudin und ein chromogenes Protein C Substrat zur Inaktivierung des Thrombins und Bestimmung der gebildeten Menge an Protein C durch Bestimmung der Farbintensität des Teststreifens enthält.

30. Testkit nach Anspruch 28 enthaltend Reagenzien für die Bestimmung der Thrombin Inaktivierung, wobei ein Reagenz Antithrombin III enthält, in dem der Teststreifen eingebracht wird, und ein Reagenz Thrombin enthält, welches anschließend zugegeben wird, und ein Reagenz ein chromogenes Thrombin-Substrat enthält, welches nach einer Inkubationszeit zur Bestimmung der verbliebenden Thrombinaktivltät Ober die Bestimmung der Farbintensität des Teststreifens zugegeben wird.

31. Ein Testkit nach mindestens einem der Ansprüche 28 bis 30, wobei anstelle eines Teststreifens eine mit Antikörpern gegen Thrombomodulin beschichtete Mikrotiterplatte verwendet wird.

32. Verwendung des Verfahrens nach Anspruch 25 zur Bestimmung des Glykosylierungsgrades von Thrombomodulin in Blut, Plasma oder Gewebe von Patienten mit Diabetes oder Homocysteinämie.

33. Verwendung des Verfahrens nach Anspruch 25 zur Bestimmung des Glykosylierungsgrades von Thrombomodulin in Blut. Plasma oder Gewebe von Patienten mit Atherosklerose.

34. Screening-Verfahren zur Bestimmung der AT III Aktivität und der Aktivität des Protein C Systems einer Probe in Anwesenheit von exogen zugesetztem Thrombomodulin humanen oder rekombinanten Ursprungs, das neben seiner Protein C aktivierenden Aktivität auch die Eigenschaft zur Beschleunigung der Inhibierung von Thrombin durch Antithrombin III aufweist, wobei bei rekombinant hergestelltem Thrombomodulin die Thrombin inaktivierende Eigenschaft durch Verknüpfung oder Zugabe eines Glykosaminoglycans wiederhergestellt wird, welches folgende Schritte einschließt:
a) zu der Probe, bevorzugterweise einer Plasmaprobe, werden folgende Reagenzien zugegeben:
i) exogenes Thrombomodulin humanen oder rekombinanten Ursprungs, das neben seiner Protein C aktivierenden Aktivität auch die Eigenschaft zur Beschleunigung der Inhibierung von Thrombin durch Antithrombin III (BITA) aufweist, bzw. dem zur Rekonstitution der BITA Eigenschaft Heparin zugesetzt wird,
ii) mindestens ein Aktivator der ohne weitere Zwischeninkubation zur Aktivierung von Prothrombin zu Thrombin führt, wobei das Prothrombin endogenes oder exogen zugesetztes Prothrombin sein kann,
iii) Phospholipide,
iv) Calciumchlorid,
v) sowie weitere Zusatzreagenzien, die zur Optimierung von Gerinnungstesten allgemein verwendet werden.
b) die Entstehung von Thrombin wird bestimmt durch Messung der Umsatzrate eines Thrombinsubstrates, wobei diese Umsatzrate durch die Meßzeit bis zur Entstehung eines Fibringerinnsels oder durch die Umsatzrate eines markierten Thrombinsubstrats ermittelt wird.

35. Verfahren nach Anspruch 34 zur selektiven Bestimmung der AT III Aktivität, **dadurch gekennzeichnet, daß** die Probe mit einem Plasma, das weniger als 5 % der normalen AT III Aktivität enthält, im Verhältnis 1:2 bis 1:20, bevorzugterweise 1:3 bis 1:5, besonders bevorzugterweise 1:4 verdünnt wird.

36. Verfahren zur Risikoabschätzung einer Thrombosegefährdung bei Diabetikern **dadurch gekennzeichnet, dass** die Glykosilierung des Thrombomodulins oder das Verhältnis der Anti-Thrombin Wirkung des Thrombomodulins zur Protein C aktivierenden Aktivität des Thrombomodulins bestimmt wird, wobei der Verlust der Anti-Thrombin Wirkung ein Indikator eines erhöhten Thromboserisikos ist

## Claims

1. A screening method for determining the anticoagulatory potential of a sample in the presence of exogenously added thrombomodulin which is of human or recombinant origin and which, in addition to its protein C-activating activity also possesses the property of accelerating the inhibition of thrombin by antithrombin III, with, in the case of recombinantly prepared thrombomodulin, the thrombin-inactivating property being restored by linking or adding the glycosaminoglycan, which method includes the following steps:
a) the following reagents are added to the sample, preferably a plasma sample:
i) exogenous thrombomodulin which is of human or recombinant origin and which can form a complex with thrombin, with this complex being able to activate the protein C in the sample, and with it being possible for the protein C to be endogenous protein C or exogenously added protein C,
ii) an activator which leads, without any further intermediate incubation, to the activation of prothrombin to thrombin, with it being possible for the prothrombin to be endogenous prothrombin or exogenously added prothrombin,
iii) phospholipids,
iv) calcium ions,
v) and also other additional reagents which are used generally for optimizing coagulation tests,
b) the reaction is started by adding the prothrombin activator-containing reagent, and
c) the formation of thrombin is determined by measuring the transformation rate of a thrombin substrate, with this transformation rate being determined by measuring the time until a fibrin clot has formed or by the transformation rate of a labeled thrombin substrate.

2. The method as claimed in claim 1, wherein the measured transformation rate is related to the transformation rate in a test assay for determining coagulation time in which no activated protein C is formed or added.

3. The method as claimed in claim 2, wherein the measured transformation rate is related to the transformation rate in a test assay which is analogous to the method as claimed in claim 1, but in which no thrombomodulin is added.

4. The method as claimed in at least one of claims 1 to 3, wherein the concentration of the activator in step a) ii) is chosen such that the coagulation time of a normal plasma in the absence of thrombomodulin is at least 20 s and at most 300 s, preferably from 30 to 150 s.

5. The method as claimed in at least one of claims 1 to 4, wherein use is made, as activators in step a) ii), of thromboplastin reagents which are known per se to the skilled person and which are of natural human or animal origin, such as from placenta, lung or brain, or are produced by recombinant means.

6. The method as claimed in claim 1, wherein the thrombomodulin is added to the sample in a separate reagent, separately from the activator-containing reagent.

7. The method as claimed in claim 1, wherein the thrombomodulin employed is of human origin.

8. The method as claimed in claim 7, wherein, in the case of recombinantly prepared thrombomodulin, the thrombin-inactivating property is restored by linking to heparin sulfate.

9. The method as claimed in claim 7, wherein, in the case of recombinantly prepared thrombomodulin, the thrombin-inactivating property is restored by adding heparin sulfate.

10. The method as claimed in at least one of claims 1 to 8, wherein the linking is effected recombinantly or synthetically.

11. The method as claimed in claim 1, wherein the quantity of thrombomodulin in the reagent is selected such that, in the presence of thrombomodulin, the coagulation times with a normal plasma are less than 300 sec, particularly preferably less than 150 sec, and wherein the difference in relation to the coagulation time without thrombomodulin is at least 40%, preferably 100 to 300% of this coagulation time without thrombomodulin.

12. The method as claimed in at least one of claims 1 to 11, wherein the thrombomodulin concentration is from 0.5 to 50 µg/ml, preferably from 1 to 10 µg/ml, based on the final volume of the test assay.

13. The method as claimed in at least one of claims 1 to 12, wherein known aggregation inhibitors are added in the test procedure in order to retard clot formation.

14. The method as claimed in one of claims 1 to 13, wherein purified coagulation factors which are not involved in the function of the protein C system or of the antithrombin III system are substituted by addition to the reagent or reagents.

15. The method as claimed in claim 14, wherein fibrinogen, factor VII, factor IX, factor X and/or prothrombin (factor II) are added to the reagent or reagents.

16. The method as claimed in claim 14, wherein antithrombin III is added in order only to detect disturbances in the protein C systems.

17. The method as claimed in claim 1, wherein, in order to selectively determine single or multiple disturbances, a solution which contains coagulation factors which are not to be codetected in the test is added to the plasma sample before it is used in the method.

18. The method as claimed in claim 17, wherein, in order to selectively diagnose the defect in or lack of a protein, the sample is prediluted, before being used in the method, in a ratio of from 1:2 to 1:20, preferably of from 1:3 to 1:5, particularly preferably of 1:9, with a plasma which contains less than 5% of this protein.

19. The method as claimed in claim 17, wherein, in order to selectively diagnose a defect in or lack of several proteins, the sample is prediluted, before being used in the method, in a ratio of from 1:2 to 1:20, preferably of from 1:3 to 1:5, particularly preferably of 1:4, with a plasma which contains less than 5% of these proteins.

20. The method as claimed in claim 17, wherein, in order to selectively diagnose anti-phospholipid antibodies, the sample is prediluted, before being used in the method, in a ratio of from 1:2 to 1:20, preferably of from 1:3 to 1:5, particularly preferably of 1:4, with an aqueous solution which contains phospholipids and/or thrombocytes at a concentration of from 0.01 to 1%(w/v).

21. The method as claimed in claim 17, wherein, in order to determine the anticoagulatory activity of antithrombin III, the sample is prediluted, in a ratio of from 1:2 to 1:10, preferably of 1:4, with an antithrombin III-deficient plasma.

22. The method as claimed in claim 1, wherein the substrate transformation rate of the sample is determined in the presence of thrombomodulin and in the absence of thrombomodulin, and this difference, or the quotient of the two values, is related to the difference or the quotient which is obtained with a normal plasma or plasma pool.

23. The use of the method as claimed in claim 1 for identifying patients who are at an increased risk of thrombosis.

24. The use of the method as claimed in claim 1 for monitoring an anticoagulation therapy.

25. A method for detecting and quantifying the glycosylation of the thrombomodulin of a patient by determining the ratio of the protein C-activating activity and the activity bringing about acceleration of the inhibition of thrombin by antithrombin III of the thromboplastin in a patient sample.

26. The method as claimed in claim 25, wherein the two activities of the thrombomodulin are determined directly in the sample.

27. The method as claimed in claim 25, wherein the endogenous thrombomodulin is isolated from the sample before the two activities are determined.

28. A test kit for use in a method as claimed in claim 25, which comprises
a) a test strip which is coated with antibodies against thrombomodulin, which strip is brought into contact with the sample,
b) a washing solution in which the incubated test strip is washed,
c) reagents for determining protein C activation
d) reagents for determining thrombin inactivation.

29. The test kit as claimed in claim 28 comprising reagents for determining protein C activation, with one reagent comprising thrombin, protein C and calcium chloride, in which the test strip is initially incubated in order to activate protein C, and a second reagent comprises antithrombin III, heparin and/or hirudin and a chromogenic protein C substrate for inactivating the thrombin and determining the quantity of protein C formed by determining the color intensity of the test strip.

30. The test kit as claimed in claim 28 comprising reagents for determining thrombin inactivation, with one reagent comprising antithrombin III, in which the test strip is introduced and one reagent comprises thrombin, which is subsequently added, and one reagent comprises a chromogenic thrombin substrate which is added, after an incubation period, for determining the remaining thrombin activity by means of determining the color intensity of the test strip.

31. The test kit as claimed in at least one of claims 28 to 30, wherein a microtiter plate coated with antibodies against thrombomodulin is used instead of a test strip.

32. The use of the method as claimed in claim 25 for determining the degree of glycosylation of thrombomodulin in blood, plasma or tissue from patients with diabetes or homocysteinemia.

33. The use of the method as claimed in claim 25 for determining the degree of glycosylation of thrombomodulin in blood, plasma or tissue from patients with atherosclerosis.

34. A screening method for determining the AT III activity and the protein C system activity of a sample in the presence of exogenously added thrombomodulin which is of human or recombinant origin and which, in addition to its protein C-activating activity, also possesses the property of accelerating the inhibition of thrombin by antithrombin III, with, in the case of recombinantly prepared thrombomodulin, the thrombin-inactivating property being restored by linking or adding the glycosaminoglycan, which method includes the following steps:
a) the following reagents are added to the sample, preferably a plasma sample:
i) exogenous thrombomodulin which is of human or recombinant origin and which, in addition to its protein C-activating activity, also possesses the property of accelerating the inhibition of thrombin by antithrombin III (AITA), or to which heparin is added in order to reconstitute the AITA property,
ii) at least one activator which leads, without any further intermediate incubation, to the activation of prothrombin to form thrombin, with it being possible for the prothrombin to be endogenous prothrombin or exogenously added prothrombin,
iii) phospholipids,
iv) calcium chloride,
v) and also other additional reagents which are used generally for optimizing coagulation tests,
b) the formation of thrombin is determined by measuring the transformation rate of a thrombin substrate, with this transformation rate being determined by measuring the time until a fibrin clot has formed or by the transformation rate of a labeled thrombin substrate.

35. The method as claimed in claim 34 for selectively determining the AT III activity, wherein the sample is diluted, in a ratio of from 1:2 to 1:20, preferably of from 1:3 to 1:5, particularly preferably of 1:4, with a plasma which contains less than 5% of the normal AT III activity.

36. A method for assessing the risk of a thrombosis threat in diabetic patients, wherein the glycosylation of the thrombomodulin, or the ratio of the antithrombin effect of the thrombomodulin to the protein C-activating activity of the thrombomodulin, is determined, with the loss of the anti-thrombin effect being an indicator of an increased risk of thrombosis.

## Revendications

1. Procède de criblage pour la détermination du potentiel anticoagulant d'un échantillon en présence de thrombomoduline humaine ou d'origine recombinante, ajoutée par addition exogène, qui, outre son activité activant la protéine C, possède également la propriété d'accélérer l'inhibition de la thrombine par l'antithrombine III, dans le cas de la thrombomoduline produite par recombinaison, la prophète inactivant la thrombine étant rétablie par liaison avec ou addition d'un glucosaminoglycane, qui comprend les étapes suivantes :
a) addition à l'échantillon de préférence à un échantillon de plasma, de réactifs suivants :
i) de la thrombomoduline d'origine humaine ou recombinante, ajoutée par addition exogène, capable de former un complexe avec la thrombine, ce complexe pouvant activer la protéine C dans l'échantillon, la protéine C pouvant être endogène ou ajoutée par addition exogène,
ii) un activateur qui, sans autre incubation intermédiaire, conduit à l'activation de la prothrombine en thrombine, la prothrombine pouvant être endogène ou ajoutée par addition exogène,
iii) des phospholipides,
iv) des ions calcium,
v) ainsi que d'autres réactifs supplémentaires qui sont en général utilisés pour l'optimisation de tests de coagulation,
b) déclenchement de la réaction par l'addition du réactif contenant un activateur de prothrombine, et
c) détermination de la formation de thrombine par mesure de la vitesse de réaction d'un substrat pour la thrombine, cette vitesse de réaction étant déterminée par le temps mesuré jusqu'à la formation d'un caillot de fibrine ou par la vitesse de réaction d'un substrat pour la thrombine marqué.

2. Procédé selon la revendication 1, dans lequel la vitesse de réaction mesurée est mise en relation avec la vitesse de réaction dans un système d'essai pour la détermination du temps de coagulation, dans lequel de la protéine C activée n'est ni formée ni ajoutée.

3. Procédé selon la revendication 2, dans lequel la vitesse de réaction mesurée est mise en relation avec la vitesse de réaction dans un système d'essai analogue au procédé selon la revendication 1, mais dans lequel de la thrombomoduline n'est pas ajoutée.

4. Procédé selon au moins l'une des revendications 1 à 3, **caractérisé en ce que** la concentration de l'activateur dans l'étape a) ii) est choisie de manière que le temps de coagulation d'un plasma normal en absence de thrombomoduline soit d'au moins 20 s et au maximum de 300 s, de préférence de 30 à 150 s.

5. Procédé selon au moins l'une des revendications 1 à 4, **caractérisé en ce qu'**on utilise comme activateurs dans l'étape a) ii) des réactifs de thromboplastine connus en soi de l'homme de métier, qui sont d'origine naturelle humaine ou animale, comme provenant du placenta, du poumon ou du cerveau, ou sont produits par des moyens de génie génétique.

6. Procède selon la revendication 1, **caractérisé en ce que** la thrombomoduline est ajoutée à l'échantillon séparément du réactif contenant l'activateur, dans un réactif spécial.

7. Procédé selon la revendication 1, **caractérisé en ce que** la thrombomoduline utilisée est d'origine humaine.

8. Procédé selon la revendication 7, **caractérisé en ce que**, dans le cas de la thrombomoduline produite par recombinaison, la propriété inactivant la thrombine est rétablie par liaison avec du sulfate d'héparine.

9. Procédé selon la revendication 7, **caractérisé en ce que**, dans le cas de la thrombomoduline produite par recombinaison, la propriété inactivant la thrombine est rétablie par addition de sulfate d'héparine.

10. Procédé selon au moins l'une des revendications 1 à 8, **caractérisé en ce que** la liaison s'effectue par recombinaison ou synthèse.

11. Procédé selon la revendication 1, **caractérisé en ce que** la quantité de thrombomoduline dans le réactif est choisie de manière qu'en présence de thrombomoduline les temps de coagulation avec un plasma normal soient inférieurs à 300 s, de façon particulièrement préférée inférieurs à 150 s, et **en ce que** la différence par rapport au temps de coagulation sans thrombomoduline est d'au moins 40 %, de préférence de 100 à 300 % de ce temps de coagulation sans thiombomoduline.

12. Procédé selon au moins l'une des revendications 1 à 11, **caractérisé en ce que** la concentration de thrombomoduline va de 0,5 à 50 µg/ml, de préférence de 1 à 10 µg/ml, par rapport au volume final du mélange d'essai.

13. Procède selon au moins l'une des revendications 1 à 12, **caractérisé en ce que**, pour ralentir la formation du caillot, on ajoute dans le procédé d'essai des inhibiteurs connus d'agrégation.

14. Procédé selon l'une quelconque des revendications 1 à 13, **caractérisé en ce que** des facteurs de coagulation purifiés, qui ne participent pas à la fonction du système protéine C ni de l'antithrombine III, sont remplacés par addition an réactif ou aux réactifs.

15. Procédé selon la revendication 14, **caractérisé en ce qu'**on ajoute au réactif ou aux réactifs du fibrinogène, du facteur VII, du facteur IX, du facteur X et/ou de la prothrombine (facteur II).

16. Procédé selon la revendication 14, **caractérisé en ce qu'**on ajoute de l'antithrombine III, afin de ne déceler que des troubles du système protéine C.

17. Procédé selon la revendication 1, **caractérisé en ce que**, pour la détermination sélective de troubles individuels ou multiples, on ajoute à l'échantillon de plasma, avant l'utilisation dans le procédé, une solution qui contient des facteurs de coagulation qui ne sont pas destinés à être déterminés dans le test.

18. Procédé selon la revendication 17, **caractérisé en ce que**, pour le diagnostic sélectif de l'anomalie ou de l'insuffisance d'une protéine, l'échantillon est prédilué, avant l'utilisation dans le procédé, avec un plasma qui contient moins de 5 % de cette protéine, en un rapport de 1:2 à 1:20, de préférence de 1:3 à 1:5, de façon particulièrement préférée de 1:4.

19. Procédé selon la revendication 17, **caractérisé en ce que**, pour le diagnostic sélectif d'une anomalie ou insuffisance de plusieurs protéines, l'échantillon est prédilué, avant l'utilisation dans le procédé, avec un plasma qui contient moins de 5% de ces protéines, en un rapport de 1:2 à 1:20, de préférence de 1:3 à 1:5; de façon particulièrement préférée de 1:4.

20. Procédé selon la revendication 17, **caractérisé en ce que**, pour le diagnostic sélectif d'anticorps anti-phospholipides, l'échantillon est prédilué, avant l'utilisation dans le procédé, avec une solution aqueuse qui contient des phospholipides et/ou des thrombocytes à une concentration de 0,01 à 1 % (p/v), en un rapport de 1:2 à 1:20, de préférence de 1:3 à 1:5, de façon particulièrement préférée de 1:4.

21. Procédé selon la revendication 17, **caractérisé en ce que**, pour la ditermination de l'activité anticoagulante de l'antitbrombine III, l'échantillon est prédilué avec un plasma déficient en antithrombine III, en un rapport de 1:2 à 1:10, de préférence de 1:4.

22. Procédé selon la revendication 1, **caractérise en ce qu'**on détermine la vitesse de réaction de l'échantillon avec le substrat en présence de thrombomoduline et en absence de thrombomoduline et on met cette différence ou le quotient des deux en relation avec la différence ou le quotient qui est obtenu avec un plasma normal ou un groupe de plasmas normaux.

23. Utilisation du procédé selon la revendication 1, pour le dépistage de patients ayant un risque élevé de thrombose.

24. Utilisation du procédé selon la revendication 1, pour la surveillance d'une thérapie d'anticoagulation.

25. Procédé pour la détection et la détermination quantitative de la glycosylation de la thrombomoduline d'un parient, par détermination du rapport de l'activité activant la protéine C et de l'activité de la thrombomoduline, provoquant l'accéleration de l'inhibition de la thrombine par l'antithrombine III, d'un échantillon provenant d'un patient.

26. Procédé selon la revendication 25, **caractérisé en ce que** la détermination des deux activités de la thrombomoduline s'effectue directement dans l'échantillon.

27. Procédé selon la revendication 25, **caractérisé en ce que** la thrombomoduline endogène est isolée à partir de l'échantillon avant la détermination des deux activités.

28. Nécessaire de test pour utilisation dans un procédé selon la revendication 25, comportant
a) une bandelette de test tapissée d'anticorps contre la thrombomoduline, qui est mise en contact avec l'échantillon,
b) une solution de lavage, dans laquelle est lavée la bandelette de test mise à incubée,
c) des réactifs pour la détermination de l'activation de la protéine C,
d) des réactifs pour la détermination de l'inactivation de la thrombine.

29. Nécessaire de test selon la revendication 28, contenant des réactifs pour la détermination de l'activation de la protéine C, un réactif contenant de la thrombine, de la protéine C et du chlorure de calcium, dans lequel la bandelette de test est d'abord mise à incuber pour l'activation de la protéine C, et un second réactif contenant de l'antithrombine III, de l'héparine et/ou de l'hirudine et un substrat chromogène pour la protéine C, pour l'inactivation de la thrombine et la détermination de la quantité formée de protéine C par détermination de l'intensité de coloration de la bandelette de test.

30. Nécessaire de test selon la revendication 28, contenant des réactifs pour la détermination de l'inactivation de la thrombine, un réactif contenant de l'antithrombine III, dans lequel est introduite la bandelette de test, et un réactif contenant de la thrombine, qui est ajouté ensuite, et un réactif contenant un substrat chromogène pour la thrombine, qui, après un temps d'incubation, est ajoute pour la détermination de l'activité résiduelle de la thrombine, par la détermination de l'intensité de coloration de la bandelette de test.

31. Nécessaire de test selon au moins l'une des revendications 28 à 30, dans lequel, au lieu d'une bandelette de test, on utilise une plaque de microtitrage tapissée d'anticorps contre la thrombomoduline.

32. Utilisation du procédé selon la revendication 25, pour la détermination du degré de glycosylation de la thrombomoduline dans le sang, le plasma ou un tissu de patients souffrant de diabète ou d'homocystéinemie.

33. Utilisation du procédé selon la revendication 25, pour la détermination du degré de glycosylation de la thrombomoduline dans le sang, le plasma ou un tissu de patients souffrant d'athérosclérose.

34. Procédé de criblage pour la détermination de l'activité AT III et de l'activité du système protéine C d'un échantillon en présence de thrombomoduline humaine ou d'origine recombinante, ajoutée par addition exogène, qui, outre son activité activant la protéine C, possède également la propriété d'accélérer l'inhibition de la thrombine par l'antithrombine III, dans le cas de thrombomoduline produite par recombinaison, la propriété inactivant la thrombine étant rétablie par liaison avec ou addition d'un glucosaminoglycane, qui comprend les étapes suivantes :
a) addition à l'échantillon, de préférence à un échantillon de plasma, des réactifs suivants :
i) de la thrombomoduline d'origine humaine ou recombinante, ajoutée par addition exogène, qui, outre son activité activant la protéine C, présente également la propriété d'accélérer l'inhibition de la thrombine par l'antithrombine III (BITA) ou on y ajoute de l'héparine pour le rétablissement de la propriété BITA,
ii) au moins un activateur qui, sans autre incubation intermédiaire, conduit à l'activation de la prothrombine en thrombine, la prothrombine pouvant être endogène ou ajoutée par addition exogène,
üi) des phospholipides,
iv) du chlorure de calcium,
v) ainsi que d'autres réactifs supplémentaires qui sont en général utilisés pour l'optimisation de tests de coagulation,
b) détermination de la formation de thrombine par mesure de la vitesse de réaction d'un substrat pour la thrombine, cette vitesse de réaction étant déterminée par le temps mesuré jusqu'à la formation d'un caillot de fibrine ou par la vitesse de réaction d'un substrat pour la thrombine marqué.

35. Procédé, selon la revendication 34, pour la détermination sélective de l'activité AT III, **caractérisé en ce que** l'échantillon est dilué avec un plasma qui contient moins de 5 % de l'activité AT III normale, en un rapport de 1:2 à 1:20, de préférence de 1:3 à 1:5, de façon particulièrement préférée de 1:4.

36. Procédé pour l'évaluation du risque de thrombose chez des diabétiques, **caractérisée en ce qu'**on détermine la glycosylation de la thrombomoduline ou le rapport de l'activité antithrombine de la thrombomoduline à l'activité activant la protéine C, la perte de l'activité antithrombine étant un indicateur d'un risque accru de thrombose.
